# EUROPEAN PATENT SPECIFICATION

(11) **EP 1 839 552 B1**
(45) Date of publication and mention of the grant of the patent: **17.08.2011**
(21) Application number: 05820060.1
(22) Date of filing: 22.12.2005
(51) Int. Cl.: A61B 1/00, G02B 23/24

(54) **ELECTRIC BENDING ENDOSCOPE DEVICE**
ELEKTRISCHES BIEGE-ENDOSKOP
ENDOSCOPE TORDU ELECTRIQUEMENT

(30) Priority: 17.01.2005 JP 2005009474
(43) Date of publication of application: 03.10.2007
(73) Proprietor: Olympus Corporation, Tokyo 151-0072 (JP)
(72) Inventor: KOITABASHI, Masanobu c/o Olympus Medical Systems Corp., Tokyo 151-0072 (JP); FURUKAWA, Tatsuya c/o Olympus Medical Systems Corp., Tokyo 151-0072 (JP)
(74) Representative: von Hellfeld, Axel
(86) International application number: PCT/JP2005/023660
(87) International publication number: WO 2006/075503

(56) References cited:
- EP-A- 0 079 525
- WO-A-96/42078
- JP-A- 2003 230 535
- JP-A- 2003 316 514
- US-A- 4 689 449
- US-A- 4 793 198
- US-A- 5 107 080

## Description

### Technical Field

The present invention relates to an electric bending endoscope device, and more particularly, relates to an electric bending endoscope device which electrically operates a bending portion which is provided at a distal end side of an insertion portion to perform a bending operation.

### Background Art

Conventionally, endoscopes have been widely used in many fields, for example, in the medical field and the industrial field. The endoscopes used in the medical field are configured to insert an elongated insertion portion into a body cavity to observe an organ in the body cavity or, if necessary, to perform various curative treatments using a treatment instrument which is inserted into a treatment instrument channel.

The endoscopes used in the industrial field are configured to insert an elongated insertion portion into the inside of various devices to observe or inspect a scratch, corrosion, or the like on the inside of a boiler, a turbine, an engine, a chemical plant, or the like.

Generally, such conventional endoscopes consecutively have a bending portion which is bendably formed at a proximal end side of a distal end portion of the insertion portion which has an elongated shape. The conventional endoscopes are configured to operate a bending operation input member such as a bending operation lever, a button, or the like provided to an operation portion to instruct and input a bending direction or a bending speed of the bending portion as a bending amount. Based on thus instructed and input information about the bending amount, a bending operation wire or the like is mechanically pulled or loosened to enable to perform the bending operation of the bending portion.

With respect to thus configured conventional endoscopes, various electric bending endoscope devices have been proposed. The electric bending endoscope devices are provided with an electric motor which is used as bending drive means in the operation portion or the like, and based on the instruction input of the bending amount information input by the bending operation input member, the devices perform a rotation control of the motor to pull or loosen the bending operation wire using the driving force of the motor. Thus, the bending operation of the bending portion can be electrically performed.

With respect to the bending operation input means in the conventional electric bending endoscope devices, for example, various types of operation members such as a push button type which has a plurality of push buttons, a joystick type, or the like can be applied. Among them, the bending operation input means of the joystick type is generally configured to perform an input operation of the bending amount information of the bending portion by tilting an axis-shaped operation element. In such a case, a tiltable range in which the operation element can be tilted corresponds to the bending amount. However, the tiltable range of the operation element is regulated in a predetermined range. Accordingly, in the case, by a slight tilting operation of the operation element, the bending amount may be largely varied.

In view of the above, for example, in an electric bending endoscope device disclosed in Japanese Unexamined Patent Application Publication No. 2003-230535, a bending operation input means of the joystick type is employed and a pouched member filled with a viscous fluid such as gel is provided as operation resistance means for generating operation resistance at the operation.

According to the device, it is expected that by providing the operation resistance means with respect to the operation element which functions as the bending operation input means of the joystick type, the tilting movement of the operation element is regulated and bending operation of the bending portion not intended by the operator can be prevented.

However, in the electric bending endoscope device disclosed in the above-described Japanese Unexamined Patent Application Publication No. 2003-230535, the pouched member filled with the viscous fluid is applied as the operation resistance means. In such a case, for example, if it is considered that the operation element of the bending operation input means is rapidly operated with a strong force or the operation is repeatedly performed, the pouched member (operation resistance means) has to be surely fixed to the inside of the bending operation input means.

Further, in the means disclosed in the above-described publication, since the operation element of the bending operation input means is configured to press the pouched member, when the pouched member is pressed, elastic resistance is generated. Accordingly, when the operation element is operated, is it difficult to burden only a viscous resistance against the viscous fluid of the inside of the pouched member as the operation resistance.

The present invention has been made in view of the above, and an object of the invention is to provide an electric bending endoscope device which has a bending operation input member having a configuration capable of obtaining desired operation resistance with a simple mechanism to contribute to improve the operability.

EP 0079525 discloses a pin at device according to the preamble of claim 1.

### Disclosure or Invention

### Means for Solving the Problem

To achieve the above object, an electric bending endoscope device according to claim 1 is provided. The electric bending endoscope device according to a first aspect of the invention includes a bending drive portion for operating an bending portion provided at a distal end side of an insertion portion to perform a bending operation, a bending operation input portion for instructing and inputting the bending operation to the bending portion, a plate-shaped member which moves in conjunction with a movement of an operation element of the bending operation input portion, a housing body which is housed to surround at least a part of the plate-shaped member and the plate-shaped member can move in the inside, and a viscous fluid sealed in the housing body.

An electric bending endoscope device according to a second aspect of the invention includes a bending operation input portion for performing an instruction input of an bending operation for operating a bending portion to perform a bending operation with respect to a bending drive portion for operating the bending portion provided at a distal end side of an insertion portion to perform the bending operation, a plate-shaped member which moves in conjunction with a movement of an operation element of the bending operation input portion, a housing body which is housed to surround at least a part of the plate-shaped member and the plate-shaped member can move in the inside, and a viscous fluid sealed in the housing body.

An electric bending endoscope device according to a third aspect of the invention includes bending drive means for operating an bending portion provided at a distal end side of an insertion portion to perform a bending

operation with respect to a bending drive portion for operating the bending portion provided at a distal end side of an insertion portion to perform the bending operation, a plate-shaped member which moves in conjunction with a movement of an operation element of the bending operation input portion, a housing body which is housed to surround at least a part of the plate-shaped member and the plate-shaped member can move in the inside, and a viscous fluid sealed in the housing body.

An electric bending endoscope device according to a third aspect of the invention includes bending drive means for operating an bending portion provided at a distal end side of an insertion portion to perform a bending operation, bending operation input means for performing an instruction input of the bending operation to the bending portion, and operation resistance means for generating operation resistance to the bending operation input means. The operation resistance means includes a plate-shaped member which moves in conjunction with a movement of an operation element of the bending operation input means, a housing body which is housed to surround at least a part of the plate-shaped member and the plate-shaped member can move in the inside, and a viscous fluid sealed in the housing body.

### Brief Description of the Drawings

Fig. 1 is a schematic configuration view illustrating a schematic configuration of an electric bending endoscope device according to a first embodiment.
Fig. 2 is a schematic configuration view illustrating a schematic configuration of an internal configuration of the electric bending endoscope device of Fig. 1.
Fig. 3 is a longitudinal sectional view illustrating a schematic configuration of a bending operation input member in an electric bending endoscope in the electric bending endoscope device of Fig. 1, in which an operation element is placed at a neutral position.
Fig. 4 is a sectional view illustrating a configuration of operation resistance means in the bending operation input member of Fig. 3 taken along the line IV - IV of Fig. 3.
Fig. 5 is an expanded sectional view of an essential portion illustrating an enlarged V portion of Fig. 3.
Fig. 6 is a longitudinal sectional view illustrating a schematic configuration of the bending operation input member in the electric bending endoscope in the electric bending endoscope device of Fig. 1, in which the operation element is tilted in a predetermined direction.
Fig. 7 is a sectional view illustrating operation resistance means in the state of Fig. 6 taken along the line VII - VII of Fig. 6.
Fig. 8 is a longitudinal sectional view illustrating a schematic configuration of a bending operation input member in an electric bending endoscope in an electric bending endoscope device according to a second embodiment.
Fig. 9 is a sectional view illustrating a configuration of operation resistance means in the bending operation input member of Fig. 8 taken along the line IX - IX of Fig. 8, in which an operation element is placed at a neutral position.
Fig. 10 is a sectional view illustrating the configuration of operation resistance means in the bending operation input member of Fig. 8 taken along the line IX - IX of Fig. 8, in which the operation element is tilted in a direction of the arrow R1.
Fig. 11 is a sectional view illustrating the configuration of the operation resistance means in the bending operation input member of Fig. 8 taken along the line IX - IX of Fig. 8, in which the operation element is tilted in a direction of the arrow R2.
Fig. 12 is a sectional view illustrating the configuration of the operation resistance means in the bending operation input member of Fig. 8 taken along the line IX - IX of Fig. 8, in which the operation element is tilted in a direction of the arrow R3.
Fig. 13 is a longitudinal sectional view illustrating a schematic configuration of a bending operation input member in an electric bending endoscope in an electric bending endoscope device according to a third embodiment.
Fig. 14 is a view illustrating a part of a configuration of operation resistance means in the bending operation input member of Fig. 13, and which illustrates a sectional view of a reception member of a housing body taken along the line XIV - XIV of Fig. 13.
Fig. 15 is a view illustrating a part of the configuration of the operation resistance means in the bending operation input member of Fig. 13 and which illustrates a plan view of a disk-shaped member viewed in a direction of the arrow E of Fig. 13.
Fig. 16 is a view illustrating a part of the configuration of the operation resistance means in the bending operation input member of Fig. 13 and which illustrates an expanded sectional view of an essential part illustrates an enlarged XVI portion of Fig. 13.
Fig. 17 is a view schematically illustrating a relationship between holes of the housing body in the bending operation input member of the electric bending endoscope of the electric bending endoscope device according to the first and third embodiments and positions of axis portions of the operation element regulated by the holes.
Fig. 18 is a view schematically illustrating a relationship between holes of the housing body in the bending operation input member of the electric bending endoscope of the electric bending endoscope device according to the second embodiment and positions of axis portions of the operation element regulated by the holes.
Fig. 19 is an external perspective view illustrating a schematic configuration of a bending operation input member in an electric bending endoscope of an electric bending endoscope device according to a fourth embodiment.
Fig. 20 is a longitudinal sectional view illustrating a schematic configuration of the bending operation input member shown in Fig. 19.
Fig. 21 is an external perspective view illustrating a schematic configuration of a bending operation input member according to a modification of the fourth embodiment.

### Best Mode for Carrying Out the Invention

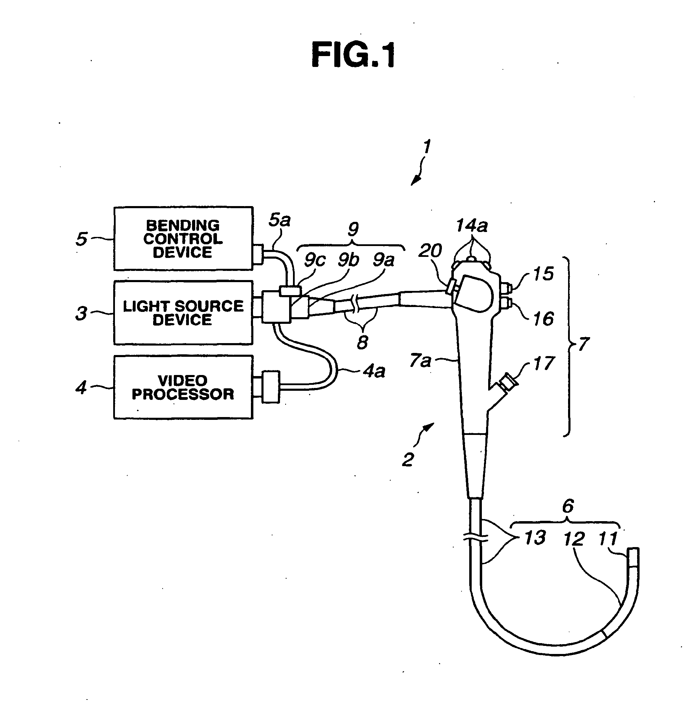

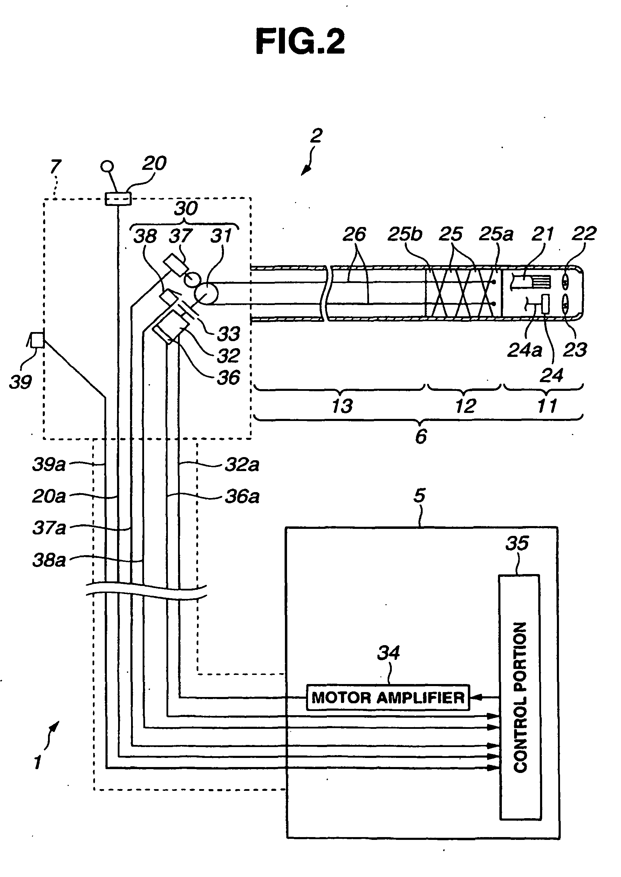

Hereinafter, descriptions of embodiments will be made with illustrated embodiments. Figs. 1 and 2 illustrate a first embodiment. Fig. 1 is a schematic configuration view illustrating a schematic configuration of an electric bending endoscope device according to the first embodiment. Fig. 2 is a schematic configuration view illustrating a schematic configuration of an internal configuration of the electric bending endoscope device of Fig. 1.

As shown in Fig. 1, an electric bending endoscope device 1 according to the first embodiment includes an electric bending endoscope 2 which has a bending drive portion 30 (see Fig. 2) which functions as bending drive means for electrically operating a bending operation of a bending portion 12 provided at a distal end side of an insertion portion 6, a light source device 3 which provides illumination light to the electric bending endoscope 2, a video processor 4 which performs a signal processing with respect to an image pickup signal or the like outputted from an image pickup apparatus 24 (see Fig. 2) which is provided in the distal end portion of the electric bending endoscope 2, and a bending control device 5 which controls drive of the bending drive portion 30 (Fig. 2) of the electric bending endoscope 2 as essential components.

The video processor 4 is connected to a monitor device (not shown), and outputs a video signal to the monitor device to display an endoscopic image.

The electric bending endoscope 2 includes the insertion portion 6 which has an elongated shape, an operation portion 7 which is consecutively provided at a proximal end side of the insertion portion 6 and also functions as a grasping portion 7a, and a universal code 8 which is extended from a side portion of the operation portion 7 as essential components.

The insertion portion 6 consecutively includes a distal end rigid portion 11 which is rigid and provided at a distal end, the bending portion 12 which is provided at a proximal end side of the distal end rigid portion 11 and is bendably formed, and a long flexible tube portion 13 which is provided at a proximal end side of the bending portion 12, and has flexibility.

The operation portion 7 includes the grasping portion 7a which is grasped and held by users. On an upper portion side of the grasping portion 7a, a plurality of video switches 14a which are used to remotely operate the video processor 4 are provided. Further, on a side surface of the grasping portion 7a, an air feed and water feed button 16 which is used to perform an air feed operation or a water feed operation, and a suction button 15 which is used to perform a suction operation is provided. To the grasping portion 7a, a bending operation input member 20 which functions as bending operation input means for performing an operation input for operating the bending portion 12 to perform a bending operation and which also functions as a bending operation input portion is provided. With respect to the bending operation input member 20, for example, a member of a joystick type or the like is applied. Further, to the grasping portion 7a, a treatment instrument insertion opening 17 which is used to insert a treatment instrument such as a biopsy forceps is provided. The treatment instrument insertion opening 17 communicates with a treatment instrument insertion channel (not shown) in the inside of the grasping portion 7a. Thus, when a treatment instrument (not shown) such as a forceps is inserted from the treatment instrument insertion opening 17, a distal end side of the treatment instrument can be protruded from a channel opening formed to the distal end rigid portion 11 through the treatment instrument insertion channel of the inside, and a biopsy or the like can be performed.

In the universal code 8, a light guide 21 or a signal cable 24a (both of them are shown in Fig. 2) are inserted. At one end portion of the universal code 8, a connector portion 9 is provided. The connector portion 9, at a distal end, includes a light guide connector (hereinafter, referred to as LG connector) 9a which can be detachably connected to the light source device 3. On a side portion of the LG connector 9a, a video connector 9b to which a connection cable 4a of the video processor 4 is detachably connected, and an angle connector 9c to which a connection cable 5a of the bending control device 5 is detachably connected are provided.

To the insertion portion 6 of the electric bending endoscope 2, as shown in Fig. 2, the light guide 21 which transmits illumination light is provided in a state that the light guide 21 is inserted through the insertion portion 6. A proximal end of the light guide 21 extends to the connector portion 9 through the operation portion 7 and inserted into the universal code 8. To a predetermined portion at a distal end side of the light guide 21, and in the distal end rigid portion 11 of the insertion portion 6, an illumination optical system 22 is fixed. When the connector portion 9 and the light source device 3 are connected with each other, illumination light emitted from a light source lamp (not shown) which is provided in the inside of the light source device 3 is transmitted to the distal end portion of the insertion portion 6. The illumination light thus transmitted to the distal end portion of the insertion portion 6 by the light guide 21 is outputted from a distal end plane of an illumination window (not shown) which is fixed to the distal end rigid portion 11 of the insertion portion 6 through the illumination optical system 22, and illuminates a subject such as an affected part.

To the insertion portion 6 of the electric bending endoscope 2, as shown in Fig. 2, the signal cable 24a which is extended from the image pickup apparatus 24 is provided in a state that the signal cable 24a is inserted through the insertion portion 6. To a predetermined portion at a distal end side of the signal cable 24a and the inside of the distal end rigid portion 11 of the insertion portion 6, the image pickup apparatus 24 which includes an image pickup device such as a CCD is fixed. In front of the image pickup apparatus 24, an image pickup optical system 23 is disposed. The signal cable 24a is inserted into the universal code 8 through the operation portion 7, and a proximal end side of the signal cable 24a is connected to the video connector 9b of the connector portion 9. Then, in a state that the video connector 9b of the connector portion 9 and the video processor 4 are connected with each other through the connection cable 4a, an output signal from the image pickup apparatus 24 is finally outputted to the video processor 4.

Thus, a reflected luminous flux (luminous flux which forms an optical subject image) of the subject which is illuminated by the illumination light outputted from the illumination window of the above-described light guide 21 is introduced through an observation window (not shown) which is adjacently provided to the illumination window. The luminous flux which is introduced from the observation window and forms the subject image reaches on a light reception plane of the image pickup apparatus 24 through an objective optical system 23. As a result, on the light reception plane of the image pickup apparatus 24, the optically formed subject image is formed.

In response to the above processing, the image pickup apparatus 24 performs a predetermined image pickup processing, that is, a photoelectric conversion processing, and outputs an image pickup signal of a predetermined form. The image pickup signal thus outputted from the image pickup apparatus 24 reaches the video connector 9b of the universal code 8 via the signal cable 24a through the operation portion 7, and further, the image pickup signal is outputted to the video processor 4 through the connection cable 4a.

The video processor 4, in response to the image pickup signal outputted from the image pickup apparatus 24 of the electric bending endoscope 2, performs a predetermined signal processing to the image pickup signal, creates a standard video signal, and then outputs the signal to a monitor device (not shown). The monitor device receives the signal and displays an endoscopic image.

At a proximal end side of the distal end rigid portion 11 of the insertion portion 6, a bending piece 25a of a most distal end of the bending portion 12 which is formed by a plurality of bending pieces 25a, 25, ..., 25b, which are rotatably connected with each other, is connected. The last piece 25b of the bending portion 12 is connected to a distal end side of the flexible tube portion 13.

Further, through the insertion portion 6, bending operation wires 26 which are used to bend the bending portion 12 in vertical and horizontal directions of an observation field are inserted. Distal end sides of the bending operation wires 26 correspond to positions corresponding to the vertical and horizontal directions of the bending portion 12, and are fixed and held by hard soldering with respect to the bending piece 25a of the most distal end respectively. Thus, the bending operation wires 26 which correspond to the respective directions are respectively pulled or loosened to bend the bending portion 12 in desired directions. In response to the operation, the distal end rigid portion 11 faces to a desired direction.

The bending operation wire 26 is pulled or loosened by an electric bending drive portion 30. That is, the bending portion 12 in the electric bending endoscope device 1 according to the embodiment is electrically bent. In Fig. 2, out of the bending operation wires 26, only two wires which are used to bend either the vertical direction or the horizontal direction are shown.

The bending drive portion 30 includes a sprocket 31 which wind a proximal end portion of the bending operation wires 26 and fixed and held to pull or loosen the bending operation wires 26, and a motor 32 which rotates the sprocket 31.

Between the sprocket 31 and the motor 32, a clutch 33 which disengages driving force of the motor 32 is provided. Thus, the bending drive portion 30 disengages the transmission of the driving force of the motor 32 by operating the clutch 33, and enables the bending portion 12 to be a so-called angle-free state.

The clutch 33 is operated under control of a control portion 35 (see Fig. 2) which is provided in the bending control device 5. Alternatively, the clutch 33 may be formed by a mechanism for manually operates the clutch 33.

A signal line 32a which is extended from the motor 32 is inserted through the universal code 8 and inserted through the connection cable 5a via an angle connector 9c, and connected to a motor amplifier 34 which is provided in the bending control device 5. Thus, a motor drive signal from the motor amplifier 34 of the bending control device 5 is transmitted to the motor 32. The motor amplifier 34 is connected to the control portion 35, and by the control portion 35, the drive is controlled.

To the motor 32, an encoder 36 which functions as rotation position detection means for detecting a rotation position is provided. A signal line 36a which is extended from the encoder 36 is inserted through the universal code 8, inserted through the connection cable 5a via the angle connector 9c, and electrically connected with the control portion 35 of the bending control device 5. Thus, a rotation position signal which indicates a rotation position of the motor 32 detected by the encoder 36 is outputted to the control portion 35.

Meanwhile, the above-described sprocket 31 is used to convert the rotational motion of the motor 32 into a forward or backward motion of the bending operation wire 26. To the sprocket 31, a potentiometer 37 which functions as rotation position detection means for detecting a rotation position is connected. A signal line 37a which is extended from the potentiometer 37 is inserted through the universal code 8, inserted through the connection cable 5a via the angle connector 9c, and electrically connected with the control portion 35 of the bending control device 5. Thus, a rotation position signal which indicates a rotation position of the sprocket 31 detected by the potentiometer 37 is outputted to the control portion 35.

In the vicinity of the clutch 33 of the bending drive portion 30, a clutch operation detection switch 38 which detects an operational state of the clutch 33, that is, an on/off state, is provided. A signal line 38a which is extended from the clutch operation detection switch 38 is inserted through the universal code 8, inserted through the connection cable 5a via the angle connector 9c, and electrically connected with the control portion 35 of the bending control device 5. Thus, a clutch operation signal which indicates an operation of the clutch 33 detected by the clutch operation detection switch 38 is outputted to the control portion 35.

Similarly, a signal line 20a which is extended from the bending operation input member 20 is inserted through the universal code 8, inserted through the connection cable 5a via the angle connector 9c, and electrically connected with the control portion 35 of the bending control device 5. Thus, a bending operation signal which indicates a bending operation inputted by the bending operation input member 20 is outputted to the control portion 35.

The control portion 35, based on the bending operation signal inputted by the bending operation input member 20, and the signals from the encoder 36 and the potentiometer 37, controls the motor amplifier 34. With the control, the motor 32 is driven and the bending operation of the bending portion 12 is performed.

In a case that the operation portion 7 is dropped during the use or unintentionally operated incorrectly, and the bending operation input member 20 is rapidly operated, in response to this, a bending operation signal is generated by the bending operation input member 20 and the bending drive portion 30 is driven. Then, a rapid bending operation of the bending portion 12 not intended by the operator is performed.

In view of the above, the electric bending endoscope 2 in the electric bending endoscope device 1 according to the embodiment has prevention means for preventing the bending operation of the bending portion 12 not intended by the operator.

That is, to the operation portion 7 of the electric bending endoscope 2, an intention detection portion 39 which detects a bending operation intention of the operator is provided. The intention detection portion 39, on the grasping portion 7a of the operation portion 7, is disposed at a portion on which a part of a palm of the operator's hand comes in contact with. Accordingly, only when the operator grasps the grasping portion of the operation portion 7, an intention detection signal is generated by the intention detection portion 39.

The intention detection portion 39 includes a sensor member which detects a motion of a hand when the operator operates the bending operation input member 20 by grasping the grasping portion 7a using very small pressure change, temperature change, vibration of the palm, or the like of the palm, for example, a pressure-sensitive sensor, a temperature sensor, a vibration sensor, or the like.

A signal line 39a which is extended from the intention detection portion 39 is inserted through the universal code 8, inserted through the connection cable 5a via the angle connector 9c, and electrically connected with the control portion 35 of the bending control device 5. Thus, an intention detection signal which indicates a bending operation intention of the operator detected by the intention detection portion 39 is outputted to the control portion 35.

The control portion 35, based on whether the intention detection signal is inputted by the intention detection portion 39 with an input instruction of the bending operation input member 20 or not, determines whether the input instruction of the bending operation input member 20 is valid or not, that is, whether the input instruction of the bending operation input member 20 is intentionally operated by the operator or not.

The bending operation input member 20 in the electric bending endoscope 2 of the electric bending endoscope device 1 according to the embodiment further includes operation resistance means which generates operation resistance to the bending operation input member 20.

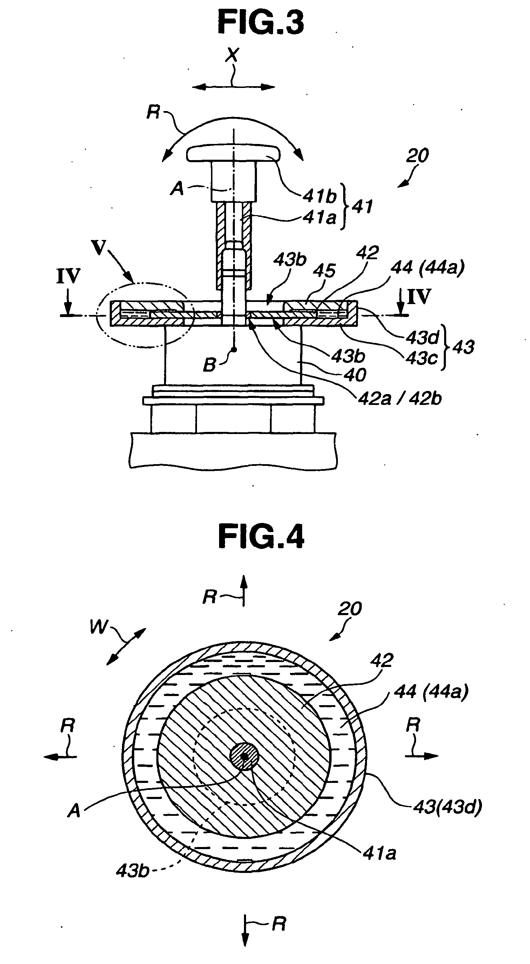

Now, a detailed configuration of the bending operation input member 20 will be described below with reference to Figs. 3 and 4.

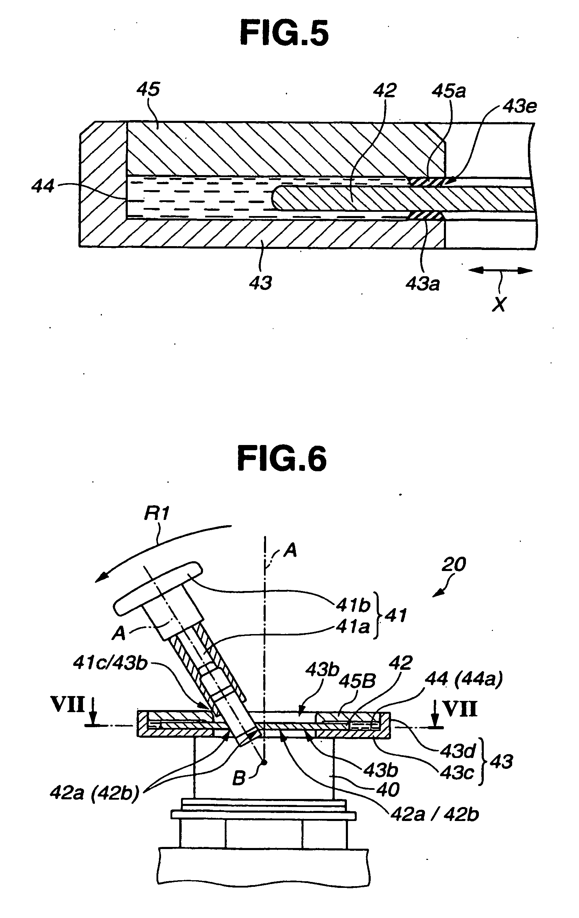

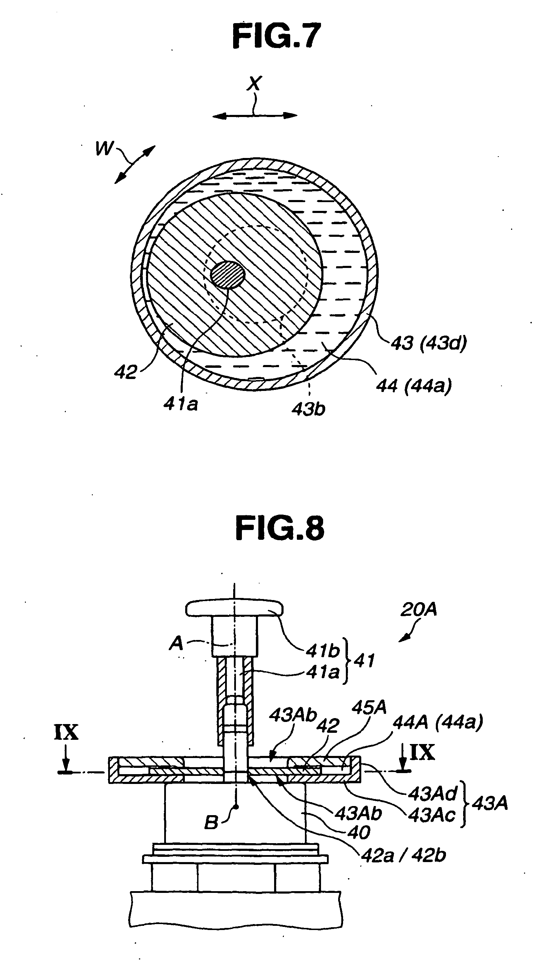

Fig. 3 is a longitudinal sectional view illustrating a schematic configuration of the bending operation input member in the electric bending endoscope in the electric bending endoscope device according to the embodiment. Fig. 4 is a sectional view illustrating a configuration of the operation resistance means in the bending operation input member of Fig. 3 taken along the line IV - IV of Fig. 3. Fig. 5 is an expanded sectional view of an essential portion illustrating an enlarged V portion of Fig. 3. Fig. 6 is, similarly to Fig. 3, a longitudinal sectional view illustrating the bending operation input member. In the drawing, the operation element of the bending operation input member in Fig. 3 is tilted in a predetermined direction. Fig. 7 is a sectional view illustrating operation resistance means in the state of Fig. 6 taken along the line VII - VII of Fig. 6. Fig. 3 illustrates a state that the operation element is at the neutral position and Fig. 6 illustrates a state that the operation element is tilted to the predetermined direction.

The bending operation input member 20 in the above-described electric bending endoscope 2 includes a main body 40, an operation element 41 which has an axis-shaped member, a neutral return spring (not shown) which maintains a neutral position of the operation element 41, and operation resistance means which uses a viscous fluid 44a as essential components.

In such a configuration, portions other than the configuration members which form the operation resistance means are similarly formed to those of conventional prevalent joystick-type bending operation input members. Accordingly, in the descriptions below, the configuration of the operation resistance means will be mainly described in detail, and the other configurations will be briefly described.

That is, the operation element 41 includes an operation element head portion 41b on which the operator's fingers come in contact with, and an axis portion 41a which has the operation element head portion 41b at the distal end and the proximal end is pivotally supported by the main body 40.

The operation element 41 is pivotally supported with a central point of the pivotal support point (see reference numeral B of Figs. 3 and 6) to be rotated within a range along a spherical surface including the arrow R direction shown in Fig. 3. In other words, the operation element 41 can be tilted with respect to an axis center line A formed when the axis portion 41a is placed at a neutral position shown in Figs. 3 and 4 within the spherical surface including the arrow R direction of Figs. 3 and 4. In a state shown in Fig. 3, that is, when the operation element 41 is placed at the neutral position, the bending portion 12 of the electric bending endoscope 2 of the electric bending endoscope device 1 according to the embodiment is in a linear state.

Further, if the operation element 41 is in a tilted state (for example, a state shown in Fig. 6), the operation element 41 can be rotated in the arrow W direction of Figs. 4 and 7 while the tilted state of the operation element 41 is maintained. Figs. 6 and 7 illustrate states that the operation element 41 is tilted at the maximum. In the states, the bending portion 12 of the electric bending endoscope 2 of the electric bending endoscope device 1 according to the embodiment is bent at the most. Accordingly, a bendable angle (hereinafter, referred to as bending angle) of the bending portion 12 of the electric bending endoscope 2 of the electric bending endoscope device 1 corresponds to the tilting angle of the operation element 41 of the bending operation input member 20.

The operation resistance means of the bending operation input member 20 includes a disk-shaped member 42 which is a plate-shaped member fixed to the axis portion 41a of the operation element 41 and movably provided in conjunction with a movement of the operation element 41, and which is formed in a substantially disk shape, a housing body (43, 45; detailed descriptions will be made below) which is formed to surround the disk-shaped member 42 such that the disk-shaped member 42 is to be movable in the inside, and the viscous fluid 44a which is sealed in the internal space of the housing body.

At substantially central portion of the disk-shaped member 42, a through hole 42a is formed, and the axis portion 41 a of the operation element 41 is inserted through the through hole 42a. An inside diameter of the through hole 42a is formed to be slightly greater than a diameter of the axis portion 41a. Between an outer periphery of the axis portion 41a and the inner periphery of the through hole 42a, an elastic seal member is provided. With respect to the member to form the disk-shaped member 42, for example, a member composed of a material which has low surface frictional resistance such as polyacetal should be applied.

On the inner periphery of the through hole 42a of the disk-shaped member 42, a seal member 42b is provided. The seal member 42b is held between the axis portion 41a of the operation element 41. Thus, the seal member 42b prevents slippage generated between the through hole 42a of the disk-shaped member 42 and the axis portion 41a of the operation element 41, and absorbs a movement difference of the axis portion 41 a which is generated when the operation element 41 is tilted.

At an outer peripheral side of the disk-shaped member 42, the housing body (43 and 45) is provided to surround at least in the vicinity of the outer peripheral edge portion of the disk-shaped member 42. The housing body includes two members, that is, a reception member 43 and a lid member 45.

The reception member 43 has an annular shape as a whole, and includes a annular portion 43c which has a seal member 43a (see Fig. 5) for supporting a bottom surface side of the outer peripheral edge portion of the disk-shaped member 42, and a standing wall portion 43d which is consecutively formed at the outer peripheral edge portion of the annular portion 43c and forms an outer peripheral wall. Thus, a cross section of the reception member 43 is formed to be a substantially L-shape.

The lid member 45 is formed by an annular plate member which has a substantially similar shape to the shape of the annular portion 43c of the reception member 43, and includes a seal member 45a for supporting an upper surface side of the outer peripheral edge portion of the disk-shaped member 42. An outer peripheral edge portion of the lid member 45 is disposed to come in contact with an upper end side inner peripheral surface of the standing wall portion 43d of the reception

Amended description page 17 for the European examination procedure member 43, and bonded and fixed at the portion. With respect to the bonding and fixing means of the reception member 43 and the lid member 45, for example, an adhesive may be used. If the reception member 43 and the lid member 45 are formed by a resin, means such as thermal welding may be used.

The shape of the housing body which is integrally formed by the reception member 43 and the lid member 45 has, as shown in Fig. 3, an internal space greater than the disk-shaped member 42 and a moving range of the disk-shaped member 42, and the shape is formed to be a substantially hollow cylindrical shape as a whole. A substantially central portion of the housing body has an annular external appearance which has a circular hole 43b. A cross section of the housing body has a channel shaped (horseshoe shaped) housing which has an opening 43e (see Fig. 5) facing toward an inner peripheral side of the annulus ring. In the inner space of the housing body, the disk-shaped member 42 is housed and the viscous fluid 44a is sealed.

On the inner peripheral edge portion of the annular portion 43c of the reception member 43 and the inner peripheral edge portion (portion in the vicinity of the opening 43e of the inner peripheral side of the annulus ring) of the lid portion 45, as shown in Fig. 5, the seal members 43a and 45a are provided to be opposed with each other. In the space formed between the seal members 43a and 45a, that is, in the opening 43e, the above disk-shaped member 42 is slidably held in a surface of the arrow X direction shown in Figs. 3 and 5 and orthogonal to the axis center line A (see Fig. 3).

In such a case, respective shapes of the seal members 43a and 45a are set such that a contact state of the respective seal members 43a and 45a and the disk-shaped member 42 becomes a point contact. That is, the shapes are set such that frictional resistance generated by the contact of the both members is reduced to be small as much as possible. Accordingly, the disk-shaped member 42 can smoothly slide in a predetermined direction without resistance with respect to the housing body (43, 45).

With such a configuration, the disk-shaped member 42 is regulated such that the member can smoothly move, within the surface orthogonal to the axis center line A in the neutral state of Fig. 3, that is, only in the direction along the surface including the arrow X direction shown in Figs. 3 and 5.

The disk-shaped member 42 can freely slide in the predetermined direction as described above, that is, in the arrow X direction (within the surface orthogonal to the axis center line A) shown in Figs. 3 and 5. Then, the disk-shaped member 42 is regulated its slide range such that the state always being held by the respective seal members 43a and 45a of the housing body (43, 45) is maintained.

In other words, the disk-shaped member 42, as described above, moves in the predetermined direction (direction along the surface including the arrow X direction of Figs. 3 and 5) in conjunction with the movement of the operation element 41. Then, if the operation element 41 is tilted in the predetermined direction (direction along the spherical surface including the arrow R direction of Figs. 3 and 4), in conjunction with the movement, the disk-shaped member 42 moves in the predetermined direction within the predetermined surface (within the surface orthogonal to the axis center line A).

In such a case, the state shown in Fig. 6, that is, in the sate that the operation element 41 is tilted at the most in the predetermined direction, a predetermined portion 41c which is in the middle of the axis portion 41a of the operation element 41 comes in contact with the inner peripheral edge portion of the hole 43b of the housing body 43. In the state, the movement of the operation element 41 and the disk-shaped member 42 is regulated. That is, the sliding range of the disk-shaped member 42 ranges from the state of Fig. 3 that the operation element 41 is in the neutral state to the state of Fig. 6 that the predetermined portion 41 c of the axis portion 41 a of the operation element 41 comes in contact with the inner peripheral edge portion of the hole 43b. In the state of Fig. 6, the state that he outer peripheral edge portion of the disk-shaped member 42 is held by the respective seal members 43a and 45a of the housing body (43, 45) is maintained.

In a housing body internal space 44 (see Fig. 5) which is the space formed by the reception member 43 and the lid member 45, the viscous fluid 44a is sealed. With respect to the viscous fluid 44a, a fluid which has viscosity, for example, oil grease or a gel-like member should be applied.

Accordingly, if the disk-shaped member 42 is moved by the operation element 41, the disk-shaped member 42 moves within the surface of the X direction such that the disk-shaped member 42 is inserted or detached with respect to the housing body internal space 44. Then, the sliding state of the disk-shaped member 42 is loosened by the operation resistance of the viscosity of the viscous fluid 44a sealed in the housing body internal space 44. Thus, even if an amount of force not intended by the operator is applied to the operation element 41 or the like, the operation element 41 and the disk-shaped member 42 are prevented from being rapidly moved.

Further, as described above, the bending operation input member 20 includes the neutral return spring (not shown). The neutral return spring is a member which urges the operation element 41 such that the predetermined neutral position of the operation element 41 is always maintained. Accordingly, for example, when the operation element 41 is tilted in the predetermined direction with application of a predetermined amount of force on the operation element 41 by the operator, if the amount of force is released, the operation element 41 always returns to the predetermined neutral position with the behavior of the neutral return spring.

Accordingly, for the viscous fluid 44a, a fluid which has viscosity enough to prevent the operation element 41 from returning to the neutral position with the neutral return spring but not inhibit the returning force is used. In other words, by setting the viscosity of the viscous fluid 44a, the returning speed of the operation element 41 to the neutral position can be set and adjusted to be a desired returning speed.

Thus configured electric bending endoscope device 1 according to the embodiment is used, as in the case shown in Fig. 1, that is, in a state that the electric bending endoscope 2 is connected to the light source device 3, the video processor 4, the bending control device 5, or the like, for example, for an endoscopy.

In such a case, the operator grasps the grasping portion 7a of the electric bending endoscope 2 to perform the endoscopy. Then, the intention detection portion 39 is covered with the operator's palm and an intention detection signal is generated. In the state, the operator performs a bending operation of the bending portion 12 by operating the bending operation input member 20 or operates various operation members provided to the operation portion 7. The control portion 35, in response to the reception of the intention detection signal from the intention detection portion 39 and the input instruction signal of the bending operation input member 20, performs a predetermined bending operation control according to the input signal based on the signals.

When the operator operates the bending operation input member 20, for example, a ball of a finger is placed on the operation element head portion 41b of the operation element 41, and the axis portion 41a is tilted in a desired direction. When the axis portion 41a is tilted, in conjunction with the axis portion 41b, the disk-shaped member 42 is moved in the housing body internal space 44. Then, since the viscous fluid 44a is sealed in the housing body internal space 44, operation resistance due to the viscosity of the viscous fluid 44a works on the disk-shaped member 42. Accordingly, the disk-shaped member 42 is moved according to the operation intention of the operator without rapid movement of the disk-shaped member 42.

Further, in a state that the axis portion 41a of the bending operation input member 20 is tilted, if the operator releases the finger, the disk-shaped member 42 returns to the neutral portion of Fig. 3 by the urging force of the neutral return spring (not shown). Also in such a case, by the operation resistance due to the viscosity of the viscous fluid 44a, the rapid movement of the disk-shaped member 42 is prevented. Accordingly, the bending operation input member 20 is gradually moved to the neutral position.

As described above, according to the above first embodiment, when the operation element 41 of the bending operation input member 20 is operated by tilting the operation element 41 or when the operation element 41 is returned from the tilted state to the neutral position, with respect to the disk-shaped member 42 which moves in conjunction with the tilt of the axis portion 41a of the operation element 41, the operation resistance due to the viscous fluid 44a works. With the configuration, when the operation element 41 of the bending operation input member 20 is operated, the rapid movement of the operation element 41 can be prevented. This also prevents rapid bending operation of the bending portion 12 of the electric bending endoscope 2 which operates in conjunction with the tilting operation of the operation element 41. Accordingly, this can contribute to improve the operability of the electric bending endoscope 2.

In the above-described first embodiment, the shape of the external appearance of the housing body internal space 44 in the bending operation input member 20 is formed to be the substantially annular shape. However, the shape is not limited to the above.

For example, in a second embodiment which will be described below, the housing body internal space 44 in the bending operation input member 20 is formed to have a substantially rectangular-shaped cross section in a case viewed from the upper surface side, and at least four corners of an inner wall are formed to have an arch shape.

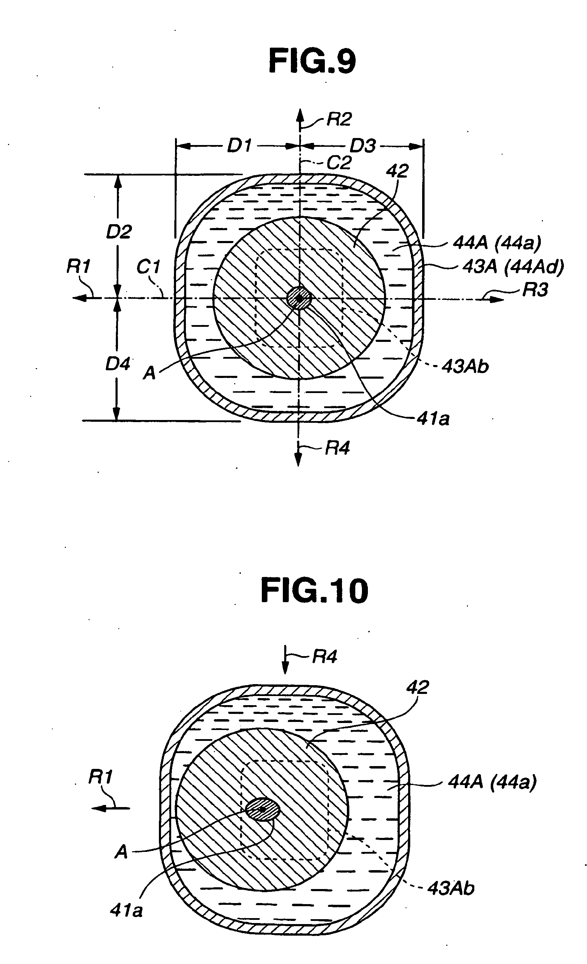

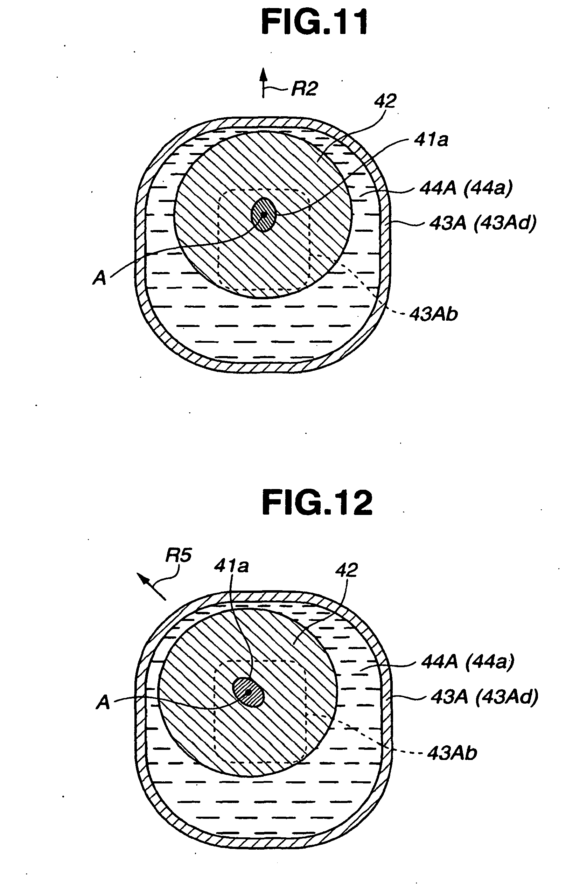

Figs. 8 to 12 illustrate the second embodiment. Fig. 8 is a longitudinal sectional view illustrating a schematic configuration of a bending operation input member in an electric bending endoscope in an electric bending endoscope device according to the embodiment. Figs. 9 to 12 are views illustrating configurations of operation resistance means in the bending operation input member of Fig. 8 taken along the line IX - IX of Fig. 8. Fig. 9 illustrates a state that an operation element is placed at a neutral position. Figs. 10 to 12 illustrate states that the operation element of the bending operation input member is tilted in respective predetermined directions. In the drawings, Fig. 10 illustrates a state that the operation element is tilted in a direction of the arrow R1, Fig. 11 illustrates a state that the operation element is tilted in a direction of the arrow R2, and Fig. 12 illustrates a state that the operation element is tilted in a direction of the arrow R3, respectively.

The basic configuration of the embodiment is substantially similar to that of the above-described first embodiment. In the embodiment, with respect to the bending operation input member 20 in the above-described first embodiment, a configuration of the housing body which constitutes a part of a bending operation input member 20A is slightly different. Accordingly, with respect to similar configurations to those of the above-described first embodiment, same reference numerals are applied, and their detailed descriptions will be omitted. Detailed descriptions about only different portions in the bending operation input member 20A will be made below.

The bending operation input member 20A in an electric bending endoscope of an electric bending endoscope device according to the embodiment, as shown in Figs. 8 and 9, includes the main body 40, the operation element 41, the neutral return spring (not shown), and the operation resistance means which uses the viscous fluid 44a as essential components.

The operation resistance means of the bending operation input member 20A includes the disk-shaped member 42 which is fixed to the axis portion 41 a of the operation element 41, a housing body (43A, 45A; detailed descriptions will be made below) which is formed to surround the disk-shaped member 42 such that the disk-shaped member 42 is to be movable in the inside, and the viscous fluid 44a which is sealed in the internal space of the housing body.

The above housing body includes two members, that is, a reception member 43A and a lid member 45A. In the members, the reception member 43A which forms a part of the housing body has a substantially similar configuration to that of the above-described first embodiment. However, as a whole, the reception member 43A is different in a point that it is formed as a substantially hollow quadrangular shape having a hole 43Ab in a substantially central portion. Then, a housing body internal space 44A which is formed by the configuration has a cross section of a substantially rectangular-shaped cross section in a case viewed from the upper surface side as shown in Fig. 9, and at least four corners of an inner wall are formed to have an arch shape. In such a case, if the cross section viewed from the upper surface side is a substantially square shape, then, functionally, the housing body internal space 44A is similar to the above-described first embodiment.

Here, as shown in Fig. 9, within the surface (that is, within the surface along directions the disk-shaped member 42 can move) orthogonal to the axis center line A of the axis portion 41 a of the operation element 41, with respect to two center lines orthogonal to the axis center line A with each other, reference numerals C 1 and C2 are given. Between the two lines, with respect to distances from the center line C1 to short sides of the housing body internal space 44A, reference numerals D2 and D4 are given respectively, and with respect to distances from the center line C2 to long sides of the housing body internal space 44A, reference numerals D1 and D3 are given respectively.

In such a case, the housing body internal space 44A is formed such that in the substantially rectangle shape having the size of the short sides = D1 + D3, and the size of the long sides = D2 + D4, at least four corners of the inner wall are formed to be an arch shape. The other configurations are substantially similar to those in the above-described first embodiment.

In thus configured above bending operation input member 20A, a tilting angle of a case that the operation element 41 is tilted, for example, in a direction of R1 of Fig. 9 to be a state shown in Fig. 10, that is, a moving amount of the disk-shaped member 42, is defined by the size D1 in the short side direction in the housing body internal space 44A.

Similarly, a tilting angle (moving amount of the disk-shaped member 42) of a case that the operation element 41 is tilted in a direction of R2 of Fig. 9 to be a state shown in Fig. 11 is defined by the size D2 in the long side direction in the housing body internal space 44A.

Further, similarly, a tilting angle (moving amount of the disk-shaped member 42) of a case that the operation element 41 is tilted in a direction of R3 of Fig. 9 is defined by the size D3 in the short side direction in the housing body internal space 44A.

Further, similarly, a tilting angle (moving amount of the disk-shaped member 42) of a case that the operation element 41 is tilted in a direction of R4 of Fig. 9 is defined by the size D4 in the long side direction in the housing body internal space 44A.

Fig. 12 illustrates a case that the operation element 41 is tilted in a direction of a reference numeral R5 of Fig. 12. Then, the tilting angle (moving amount of the disk-shaped member 42) is similarly defined by the size in the tilting direction in the housing body internal space 44A.

As described above, in the bending operation input member 20A according to the embodiment, by varying the setting of the shape of the cross section of the housing body internal space 44A, that is, settings of respective sizes of D1, D2, D3, and D4, different bending angles can be set depending on the bending directions. In the embodiment, for example, among the above-described respective sizes, the sizes are set such that the size D1 = D3, and the size D2 = D4, and the size D1 < D2. Thus, in the embodiment, the bending angle of a case that the operation element 41 is tilted in the direction of the arrow R1 (for example, bending in the left direction) of Fig. 9 is set to be equal to the bending angle of a case that the operation element 41 is tilted in the direction of the arrow R3 (for example, bending in the right direction) of Fig. 9. Further, the bending angle of a case that the operation element 41 is tilted in the direction of the arrow R2 (for example, bending in the upward direction) of Fig. 9 is set to be equal to the bending angle of a case that the operation element 41 is tilted in the direction of the arrow R4 (for example, bending in the downward direction) of Fig. 9. Further, the bending angle (bending angle in the horizontal direction) of a case that the operation element 41 is tilted in the direction of the arrow R1 or the direction of the arrow R3 of Fig. 9 is set to be different from the bending angle (bending angle in the vertical direction) of a case that the operation element 41 is tilted in the direction of the arrow R2 or the direction of the arrow R4 of Fig. 9.

Further, in addition to the above, among the above-described respective sizes, by setting the size D1 ≠ D3, and the size D2 ≠ D4, respective bending angles in the horizontal direction and the vertical direction can be set to any angles.

As described above, in the above-described second embodiment, similar advantages to those in the above-described first embodiment can be obtained. At the same time, by varying the settings of the respective sizes in the shape of the cross section of the housing body internal space 44A to regulate the moving amount of the disk-shaped member 42 according to the tilting angle of the operation element 41, the bending operation input member 20A can be set such that the bending angle of the bending portion (12) is set to a desired angle for the usage. Accordingly, this can contribute to improve the operability of the electric bending endoscope.

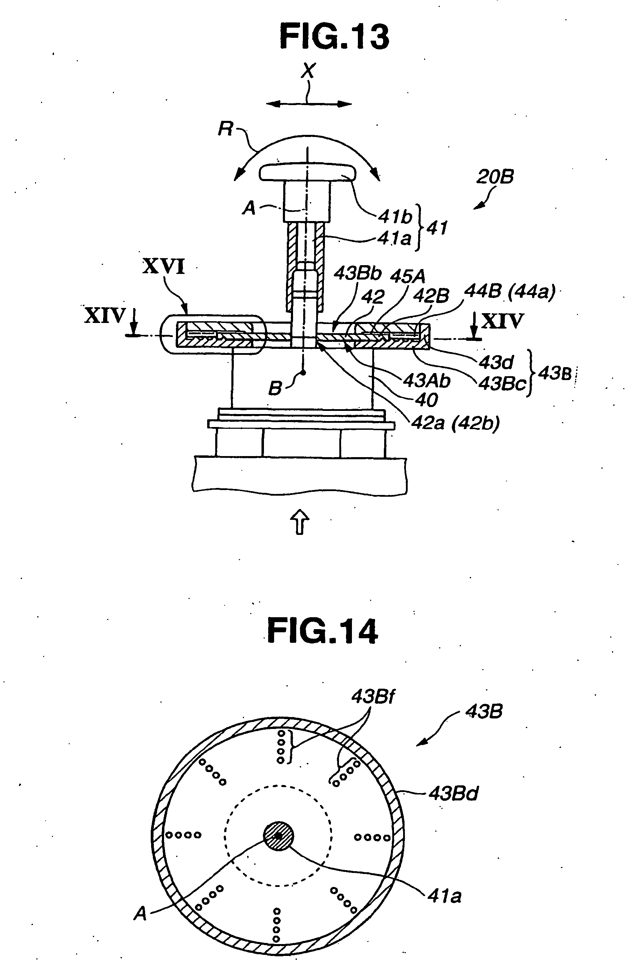

Next, with respect to an electric bending endoscope device according to a third embodiment, descriptions will be made with reference to Figs. 13 to 16.

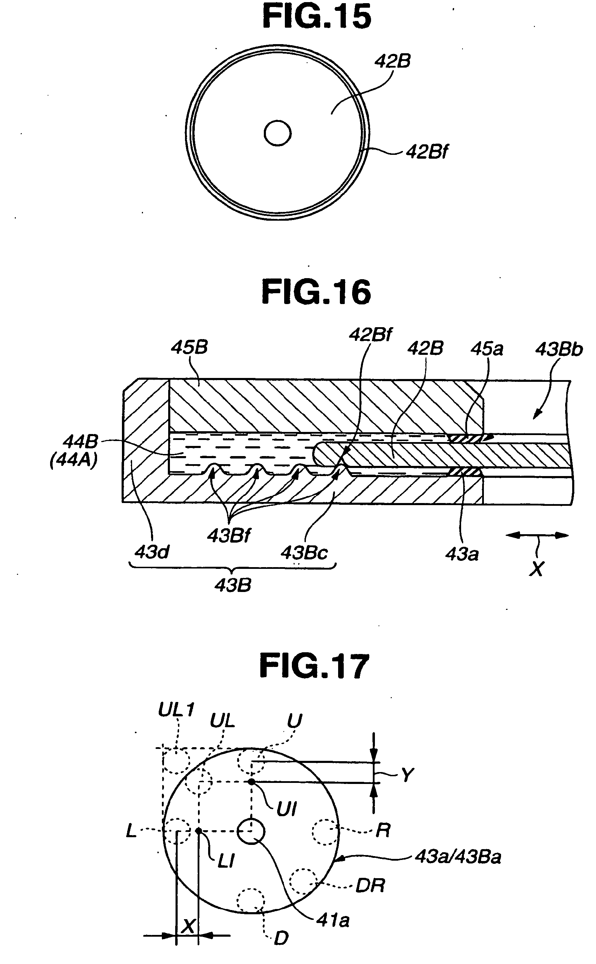

Figs. 13 to 16 illustrate the third embodiment of the present invention. Fig. 13 is a longitudinal sectional view illustrating a schematic configuration of a bending operation input member in an electric bending endoscope in an electric bending endoscope device according to the embodiment. Figs. 14 to 16 are views illustrating parts of a configuration of operation resistance means in the bending operation input member of Fig. 13. Fig. 14 is a sectional view of a reception member of a housing body taken along the line XIV - XIV of Fig. 13. Fig. 15 is a plan view illustrating a disk-shaped member viewed in a direction of the arrow E of Fig. 13. Fig. 16 is an expanded sectional view of an essential part illustrates an enlarged XVI section of Fig. 13.

The basic configuration of the embodiment is substantially similar to that of the above-described first embodiment. In the embodiment, with respect to the bending operation input member 20 in the above-described first embodiment, a configuration of a housing body which constitutes a part of a bending operation input member 20B is slightly different. Accordingly, with respect to the similar configurations to those of the above-described first embodiment, same reference numerals are applied, and their detailed descriptions will be omitted. Detailed descriptions about only different portions in the bending operation input member 20B will be made below.

The bending operation input member 20B in an electric bending endoscope of an electric bending endoscope device according to the embodiment, as shown in Fig. 13, includes the main body 40, the operation element 41 which has a lever shape tiltable in a plurality of directions with a reference of a predetermined neutral position, the neutral return spring (not shown), and the operation resistance means which uses the viscous fluid 44a as essential components.

The operation resistance means of the bending operation input member 20B includes a disk-shaped member 42B which is fixed to the axis portion 41a of the operation element 41, housing body (43B, 45B; detailed descriptions will be made below) which is formed to surround the disk-shaped member 42B such that the disk-shaped member 42B is to be movable in the inside, and the viscous fluid 44a which is sealed in the internal space of the housing body.

The above housing body includes two members, that is, a reception member 43B and a lid member 45B. In the members, the reception member 43B has a substantially similar configuration to that of the above-described first embodiment. However, the reception member 43B is different in a point that in an inner surface side of the annular portion 43Bc of the reception member 43B, a plurality of protrusion portions 43Bf formed by radially arranging the protrusion portions 43Bf with a predetermined space are formed. Further, corresponding to the portions, or the disk-shaped member 42B, on a surface opposed to the above-described protrusion portions 43Bf at a portion closer to an outer peripheral edge, a v-shaped peripheral groove (hereinafter, referred to as V-shaped groove) 42Bf is formed.

Then, in a state that the bending operation input member 20B is assembled, as shown in Fig. 16, based on the tilting operation (see the direction of the arrow R of Fig. 13) of the operation element 41, the disk-shaped member 42B is moved to a predetermined direction (in the direction of the arrow X of Figs. 13 and 16), that is, a direction substantially orthogonal to the axis direction of the operation element 41 in a state of being disposed at the neutral position. In such a case, the plurality of protrusion portions 43Bf move step by step with respect to the V-shaped groove 42Bf by repeating engaging and detaching. Accordingly, each time the protrusion portions 43Bf engage into the V-shaped groove 42Bf, operation resistance to the movement in the arrow X direction of the disk-shaped member 42B is generated. Further, in a housing body internal space 44B of the housing body, similarly to the above-described respective embodiments, the viscous fluid 44a is sealed.

The protrusion portions 43Bf are formed at portions correspond to the V-shaped groove 42Bf of the disk-shaped member 42B when the disk-shaped member 42B is housed in the inside of the housing portion. Then, when the disk shaped member 42B is moved in a predetermined direction (X direction of Fig. 16) in conjunction with the tilting operation of the operation element 41, the protrusion portions 43Bf are inserted into or detached from the V-shaped groove 42Bf.

As described above, with the protrusion portions 43Bf and the V-shaped groove 42Bf, a so-called click mechanism is formed. In such a case, the engaging force between the protrusion portions 43Bf and the V-shaped groove 42Bf may be set at a degree to give the operator a feel of click when the protrusion portions 43Bf are engaged to or detached from and the V-shaped groove 42Bf.

Then, when the operator releases the hand in the state that the operation element 41 is tilted, the operation element 41 starts to move to the neutral position by the urging force of the neutral return spring. In conjunction with this, the disk-shaped member 42B moves to the neutral position against the operation resistance of the viscous fluid 44a. When the protrusion portions 43Bf engage into the V-shaped groove 42Bf during the movement, the movement is not stopped due to the engagement, and the operation element 41 (disk-shaped member 42Bf) can finally be returned to the predetermined neutral position. The other configurations are substantially similar to those in the above-described first embodiment.

In the thus configured above-described bending operation input member 20B, in addition to the operation resistance due to the viscous fluid 44a sealed in the housing body internal space 44B, the operation resistance which is generated by the engagement of the protrusion portions 43Bf and the V-shaped groove 42Bf is given as a load when the operation element 41 is in the tilting operation or the returning operation.

As described above, in the above third embodiment, similar advantages to those in the above-described first embodiment can be obtained. At the same time, in addition to the operation resistance due to the viscous fluid 44a, further, the operation resistance due to the mechanical operation is generated in conjunction with the operation performed in the tilting operation or the returning operation of the operation element 41. Accordingly, when the operation element 41 is operated, a more fine operation can be performed. Thus, this can contribute to improve the operability of the electric bending endoscope.

In the embodiment, the plurality of protrusion portions 43Bf is provided. However, at least one protrusion portion may be provided. If, the position where the feel of click generated by providing the protrusion portions 43 Bf is generated is set to correspond to the predetermined bending angle, at the time the operator can recognize the feel of click, the operator can readily check the predetermined bending angle of the bending portion (12) to be set during the operation.

Further, in addition to the configuration of the embodiment, a form which also includes the configuration of the above-described second embodiment may be employed. In such a case, instead of the V-shaped groove of the peripheral groove, a groove portion which has a predetermined arch length or a linear-shaped groove may be employed. Then, it may be formed that by forming a protrusion portion at a portion corresponding to the groove, in respective directions of a direction the moving amount of the disk-shaped member 42B becomes large and a direction the moving amount becomes small, the operation resistance generated by the V-shaped groove and the protrusion portion are ensured.

In thus configured case, in addition the advantages similar to those in the third embodiment, the above-described advantages similar to those in the second embodiment can be obtained.

In the above-described respective embodiments, the ranges that the operation element 41 of the bending operation input members (20, 20A, 20B) can be tilted are regulated by the shapes of the holes 43b, 43Ab, and 43Bb which are formed on the housing bodies (43, 43A, 43B, and 45, 45A, 45B).

For example, in the above-described first and third embodiments, the holes 43b and 43Bb are formed in the substantially circular shapes. In the above-described second embodiment, the hole 43Ab is formed in the substantially quadrangular shape (the shape which has four substantially rectangular-shaped arch-shaped corners of the inner wall).

By tilting the operation element 41, the bending angle of the bending portion (12; see Fig. 1) is set. In such a case, if the holes of the housing body are formed in the substantially circular shape, it becomes the figure shown in Fig. 17.

Fig. 17 is a view schematically illustrating a relation between the holes of the housing body and the positions of the axis portion of the operation element which are regulated by the holes.

In Fig. 17, in a case that the operation element 41 is tilted, respective positions of the axis portion 41a which are regulated by the holes 43b are indicated by reference numerals U, D, L, and R, and reference numerals UL, and DR. In the case, when the axis portion 41 a of the operation element 41 is placed at the reference numeral U, the bending portion (12) is in a maximum bending state in the upward direction (up).

When the axis portion 41 a of the operation element 41 is placed at the reference numeral D, the bending portion (12) is in a maximum bending state in the downward direction (down).

When the axis portion 41 a of the operation element 41 is placed at the reference numeral L, the bending portion (12) is in a maximum bending state in the leftward direction.

When the axis portion 41a of the operation element 41 is placed at the reference numeral R, the bending portion (12) is in a maximum bending state in the rightward direction.

Further, when the axis portion 41a of the operation element 41 is placed at the reference numeral UL, the bending portion (12) is in a bending state in the leftward and obliquely upward direction.

Similarly, when the axis portion 41 a of the operation element 41 is placed at the reference numeral DR, the bending portion (12) is in a bending state in the rightward and obliquely downward direction.

Here, for example, if an operation that the axis portion 41 a of the operation element 41 is placed at the reference numeral UL, that is, a twist operation is performed, an actual position to ensure the maximum bending state in the upward direction and the leftward direction is obtained when the axis portion 41 a is moved to the position indicated by a reference numeral UL1 of Fig. 17.

However, as in the cases of the above-described first and third embodiments, when the holes 43b and 43Bb of the housing body are formed in the substantially circular shape, the movable range of the axis portion 41 a is regulated. Thus, if the twist operation is performed, the axis portion 41 a of the operation element 41 cannot be moved to the position of the reference numeral UL1. Accordingly, in the configuration, the bending portion (12) may not be bent to the maximum bending state (position of the reference numeral UL1) in each direction.

Specifically, for example, in Fig. 17, when the axis portion 41a of the operation element 41 is placed at the position of the reference numeral UL, in the upward direction, the bending angle is set to the angle corresponding to the angle formed when the axis portion 41a is placed at the reference numeral U1, and in the leftward direction, the bending angle is set to the angle corresponding to the angle formed when the axis portion 41a is placed at the reference numeral L1, respectively. Accordingly, when the axis portion 41a is placed at the position of the reference numeral UL, the bending angle is regulated to be smaller than the maximum bending angles corresponding to the positions in the respective directions shown by the reference numeral U and L by the amounts shown by reference numerals Y and X. In Fig. 17, if the axis portion 41 a of the operation element 41 is placed at the position of the reference numeral DR, it is just similar to the above.

Depending on the electric bending endoscope device to be used, also in a case that an operation (in the direction of the reference numerals UL, DR, or the like) in an oblique direction is performed in the twist operation, the configuration that the bending angle in each direction (directions of the reference numerals U, D, L, R) becomes a similar bending angle to the maximum bending state may be preferred.

Then, similar to the configurations described in the above-described second embodiment, if the hole 43Ab of the housing body is formed in a substantially quadrangular shape (shape which has four substantially rectangular-shaped arch-shaped corners of the inner wall), when the operation is performed in the oblique direction (in the direction of the reference numerals UL, DR, or the like) in the twist operation, the maximum bending state can be obtained in each direction.

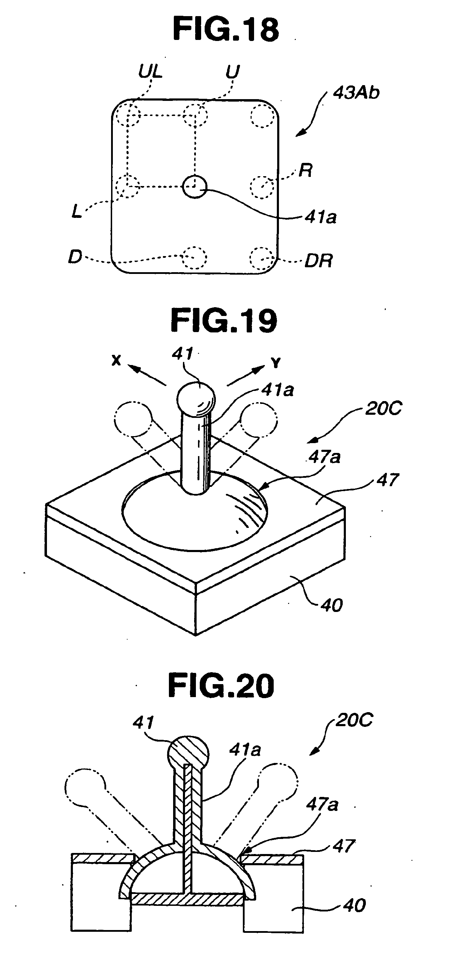

In such a case, the hole 43Ab of the housing body becomes the form shown in Fig. 18. That is, when the axis portion 41 a of the operation element 41 is placed at the position of the reference numeral UL, in the upward direction, a bending angle (maximum bending angle in the upward direction) corresponding to an angle formed when the axis portion 41a is placed at the reference numeral U, and in the leftward direction, a bending angle (maximum bending angle in the leftward direction) corresponding to an angle formed when the axis portion 41 a is placed at the reference numeral L, are respectively set. Accordingly, when the axis portion 41a is placed at the position of the reference numeral UL of Fig. 18, a bending angle similar to the maximum bending angles in the respective directions shown by the reference numerals U and L of the drawing is set. In Fig. 18, if the axis portion 41 a of the operation element 41 is placed at the position of the reference numeral DR, it is just similar to the above.

With the configuration, when the operation is performed in the oblique direction (in the direction of the reference numerals UL, DR, or the like) in the twist operation, the maximum bending state can always be obtained. Next, with respect to an electric bending endoscope device according to a fourth embodiment of the present invention, descriptions will be made with reference to Figs. 19 to 21.

Figs. 19 and 20 illustrate the fourth embodiment. Fig. 19 is an external perspective view illustrating a schematic configuration of a bending operation input member in an electric bending endoscope of an electric bending endoscope device according to the embodiment. Fig. 20 is a longitudinal sectional view illustrating a schematic configuration of the bending operation input member of Fig. 19.

The basic configuration of the embodiment is substantially similar to that of the above-described first embodiment. In the embodiment, with respect to the bending operation input member 20 in the above-described first embodiment, the configuration is different in that a regulation member 47 which can regulate the movement of the operation element 41 to set the tilting angle is further provided. Accordingly, with respect to similar configurations to those of the above-described first embodiment, same reference numerals are applied, and their detailed descriptions will be omitted. Detailed descriptions about only different portions in a bending operation input member 20C will be made below. In Fig. 20, a simplified internal configuration of the bending operation input member 20C is shown.

The bending operation input member 20C in the electric bending endoscope of the electric bending endoscope device according to the embodiment, as shown in Figs. 19 and 20, includes the main body 40, the operation element 41, the neutral return spring (not shown), and the operation resistance means which uses the viscous fluid (44a; not shown) as essential components. In the embodiment, in addition to them, the regulation member 47 which can regulate the movement of the operation element 41 to set the tilting angle is further provided on the upper surface of the main body 40.

The regulation member 47 is, for example, a laminated member which can cover the upper surface of the main body 40, and at a substantially central portion, a hole 47a for regulating the movement of the operation element 41 is formed.

If the operation element 41 is tilted from the neutral position toward a direction, for example, the direction of the arrow X, the arrow Y, or the like of Fig. 19, a predetermined portion of the axis portion 41a of the operation element 41 comes in contact with an inner peripheral edge portion of the hole 47a of the regulation member 47. Thus, the movement of the operation element 41 is regulated, and the tilting operation is regulated at a predetermined angle.

Then, by setting the size of the hole 47a to any size, the tilting angle of the operation element 41 can be set. Accordingly, the bending angle of the bending portion (not shown, see Fig. 1) can be set to a desired angle. The other configurations are similar to those in the above-described first embodiment.

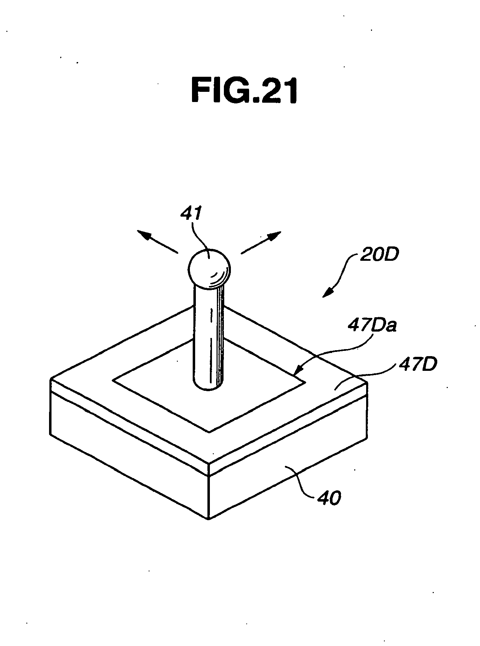

Fig. 21 illustrates a modification of the fourth embodiment, and is an external perspective view illustrating a schematic configuration of a bending operation input member.

In the modification, as shown in Fig. 21, a shape of the hole 47Da which is formed on a regulation member 47D to be provided to the main body 40 of a bending operation input member 20D is formed to be a substantially rectangular shape. The other configurations are similar to those in the above-described fourth embodiment.

In the modification shown in Fig. 21, as described using the above-described Fig. 18, when the tilting operation of the operation element 41 is performed in the oblique direction (in the direction of the reference numerals UL, DR, or the like) in the twist operation, the maximum bending state can always be obtained.

As described above, according to the above-described fourth embodiment and the modification of the fourth embodiment, in the bending operation input members 20C and 20D, by providing the regulation members 47 and 47D which regulate the movement of the operation element 41 and set the tilting angles, the bending portion of the electric bending endoscope can be set to a desired bending angle.

In the above-described fourth embodiment and the modification of the fourth embodiment, the regulation members 47 and 47D may be detachably configured with respect to the main body 40 of the bending operation input members 20C and 20D.

In such a case, by providing a plurality of the regulation members 47 and 47D which have different sizes of the hole 47a, the setting of the bending angle of the bending portion corresponding to the use of the electric bending endoscope to which the bending operation input member 20C or the bending operation input member 20D is applied can be set to any angle just by replacing the regulation member 47 or the regulation member 47D.

As described above, according to the present invention, by providing the bending operation input member which has the configuration capable of obtaining a desired operation resistance with the simple mechanism, the electric bending endoscope device which can contribute to improve the operability can be provided.

## Claims

1. An electric bending endoscope device (1) comprising:
a bending drive portion (30) for operating an bending portion (12) provided at a distal end side of an insertion portion (6) to perform a bending operation;
a bending operation input portion (20, 20B) for performing an instruction input of a bending operation to the bending drive portion (30);
**characterized by** further comprising:
a plate-shaped member (42, 42B) which moves in conjunction with a movement of an operation element (41) of the bending operation input portion (20, 20B);
a housing body (43, 43A, 43B, 45, 45A, 45B) which is formed to surround at least a part of the plate-shaped member and to house (42, 42B) such that the plate-shaped member (42, 42B) can move In the inside;
a viscous fluid (44a) sealed in the housing body;
a groove portion (42Bf) formed to a predetermined portion of the plate-shaped member (42, 42B) close to the outer peripheral edge of the plate-shaped member (42, 42B); and
a protrusion portion (43Bf, 43b, 43Ab, 43Bb) formed on the housing body (43, 43A, 43B, 45, 45A, 45B), wherein, when the plate-shaped member (42, 42B) moves in conjunction with the movement of the operation element (41) of the bending operation input portion (20, 20B), the protrusion portion (43Bf, 43b, 43Ab, 43Bb) is inserted into or detached from the groove portion (42Bf);
wherein the protrusion portion (43Bf, 43b, 43Ab, 43Bb) is formed at a portion corresponding to the groove portion (42Bf) of the plate-shaped member (42, 42B) when the plate-shaped member (42, 42B) is housed In the inside of the housing portion(43, 43A, 43B, 45, 45A, 45B) in a predetermined state.

2. The electric bending endoscope device according to claim 1, wherein the plate-shaped member (42, 42B) is formed in a substantially disk shape.

3. The electric bending endoscope device according to claim 2,
wherein the housing body (43, 43A, 43B, 45, 45A, 45B) has a hollow and substantially cylindrical shape having an Internal space (44) greater than the plate-shaped member (42, 42B) and a moving range of the plate-shaped member (42, 42B), and
wherein in the Internal space (44), the plate-shaped member (42, 42B) is housed and the viscous fluid (44a) is sealed.

4. The electric bending endoscope device according to claim 2,
wherein the housing body (43, 43A, 43B, 45, 45A, 45B) has a hollow and substantially quadrangular shape having an internal space (44A) greater than the plate-shaped member (42, 42B) and a moving range of the plate-shaped member (42, 42B), and
wherein in the internal space (44A), the plate-shaped member (42, 42B) is housed and the viscous fluid is sealed (44a).

5. The electric bending endoscope device according to one of claims 1-4, wherein the bending operation input portion (20, 20B) is a joystick type.

6. The electric bending endoscope device according to claim 5, further comprising: a regulation member (47, 47D) for regulating the movement of the operation element (41) of the bending operation input portion (20, 20B).

7. The electric bending endoscope according to claim 1,
wherein the operation element (41) of the bending operation input portion (20, 20B) has a lever shape tiltable in a plurality of directions with a reference of a predetermined neutral position, and
the plate-shaped member (42, 42B) is provided in a direction substantially orthogonal to an axis direction (41a) of the operation element (41) in the state being disposed at the neutral position such that the plate-shaped member (42, 42B) can move in the plurality of directions in conjunction with the movement of the operation element (41).

## Patentansprüche

1. Ein elektrisches Biegeendoskop (1), aufweisend:
einen Biegeantriebsabschnitt (30) zum Betätigen eines Biegeabschnitts (12), der an einer distalen Endseite eines Einführabschnitts (6) angeordnet ist, um einen Biegevorgang durchzuführen;
einen Biegevorgangeingabeabschnitt (20, 20B) zur Durchführung einer Anweisungseingabe für einen Biegevorgang an den Biegeantriebsabschnitt (30);
**gekennzeichnet durch** weiterhin aufweisend:
ein plattenförmiges Bauteil (42, 42B), das sich zusammen mit einer Bewegung eines Betätigungselements (41) des Biegevorgangeingabeabschnitts (20, 20B) bewegt;
einen Aufnahmekörper (43, 43A, 43B, 45, 45A, 45B), der so ausgebildet ist, dass er zumindest einen Teil des plattenförmigen Bauteils umgibt und dieses so aufnimmt (42, 42B), dass sich das plattenförmige Bauteil (42, 42B) in seinem Inneren bewegen kann;
ein viskoses Fluid (44a), das in dem Aufnahmekörper eingeschlossen ist;
einen Vertiefungsabschnitt (42Bf), der in einem bestimmten Abschnitt des plattenförmigen Bauteils (42, 42B) nahe der äußeren Umfangskante des plattenförmigen Bauteils (42, 42B) ausgebildet ist; und
einen vorspringenden Abschnitt (43Bf, 43b, 43Ab, 43Bb), der in dem Aufnahmekörper (43, 43A, 43B, 45, 45A, 45B) ausgebildet ist, wobei, wenn sich das plattenförmige Bauteil (42, 42B) zusammen mit der Bewegung des Betätigungselements (41) des Biegevorgangeingabeabschnitts (20, 20B) bewegt, der vorspringende Abschnitt (43Bf, 43b, 43Ab, 43Bb) in den Vertiefungsabschnitt (42Bf) eingesetzt oder hiervon getrennt wird; wobei
der vorspringende Abschnitt (43Bf, 43b, 43Ab, 43Bb) an einem Abschnitt entsprechend dem Vertiefungsabschnitt (42Bf) des plattenförmigen Bauteils (42, 42B) ausgebildet ist, wenn das plattenförmige Bauteil (42, 42B) in einer bestimmten Lage im Inneren des Aufnahmekörpers (43, 43A, 43B, 45, 45A, 45B) aufgenommen ist.

2. Das elektrische Biegeendoskop nach Anspruch 1, wobei das plattenförmige Bauteil (42, 42B) im Wesentlichen scheibenförmig ausgebildet ist.

3. Das elektrische Biegeendoskop nach Anspruch 2,
wobei der Aufnahmekörper (43, 43A, 43B, 45, 45A, 45B) eine hohle und im Wesentlichen zylindrische Form mit einem Innenraum (44) hat, der größer als das plattenförmige Bauteil (42, 42B) und als ein Bewegungsbereich des plattenförmigen Bauteils (42, 42B) ist; und
wobei in dem Innenraum (44) das plattenförmige Bauteil (42, 42B) aufgenommen und das viskose Fluid (44a) eingeschlossen ist.

4. Das elektrische Biegeendoskop nach Anspruch 2,
wobei der Aufnahmekörper (43, 43A, 43B, 45, 45A, 45B) eine hohle und im Wesentlichen viereckige Form mit einem Innenraum (44A) hat, der größer als das plattenförmige Bauteil (42, 42B) und als ein Bewegungsbereich des plattenförmigen Bauteils (42, 42B) ist; und
wobei in dem Innenraum (44A) das plattenförmige Bauteil (42, 42B) aufgenommen und das viskose Fluid (44a) eingeschlossen ist.

5. Das elektrische Biegeendoskop nach einem der Ansprüche 1 bis 4,
wobei der Biegevorgangeingabeabschnitt (20, 20B) vom Joystick-Typ ist.

6. Das elektrische Biegeendoskop nach Anspruch 5, weiterhin aufweisend: ein Regulierbauteil (47, 47D) zum Regulieren der Bewegung des Betätigungselements (41) des Biegevorgangeingabeabschnitts (20, 20B).

7. Das elektrische Biegeendoskop nach Anspruch 1,
wobei das Betätigungselement (41) des Biegevorgangeingabeabschnitts (20, 20B) eine Hebelform hat und bezüglich einer bestimmten Neutrallage als Referenz in eine Mehrzahl von Richtungen verkippbar ist; und
das plattenförmige Bauteil (42, 42B) in einer Richtung im Wesentlichen senkrecht zu einer Achsenrichtung (41a) des Betätigungselements (41) angeordnet ist, wenn dieses in der Neutrallage ist, so dass sich das plattenförmige Bauteil (42, 42B) zusammen mit einer Bewegung des Betätigungselements (41) in die Mehrzahl von Richtungen bewegen kann.

## Revendications

1. Dispositif endoscopique tordu électriquement (1) comprenant :
une partie d'entraînement de torsion (30) destinée à actionner une partie de torsion (12) prévue au niveau d'un côté d'extrémité distale d'une partie d'insertion (6) pour réaliser une opération de torsion ;
une partie d'entrée d'opération de torsion (20, 20B) destinée à réaliser une entrée d'instruction d'une opération de torsion sur la partie d'entraînement de torsion (30) ;
**caractérisé en ce qu'**il comprend en outre :
un élément en forme de plaque (42, 42B) qui se déplace en liaison avec un mouvement d'un élément d'actionnement (41) de la partie d'entrée d'opération de torsion (20, 20B) ;
un corps de logement (43, 43A, 43B, 45, 45A, 45B) qui est formé pour entourer au moins une partie de l'élément en forme de plaque et le loger (42, 42B) d'une manière telle que l'élément en forme de plaque (42, 42B) peut se déplacer à l'intérieur ;
un fluide visqueux (44a) contenu de manière étanche dans le corps de logement ;
une partie de rainure (42Bf) formée sur une partie prédéterminée de l'élément en forme de plaque (42, 42B) proche du bord périphérique externe de l'élément en forme de plaque (42, 42B) ; et
une partie de saillie (43Bf, 43b, 43Ab, 43Bb) formée sur le corps de logement (43, 43A, 43B, 45, 45A, 45B), dans laquelle, lorsque l'élément en forme de plaque (42, 42B) se déplace en liaison avec le mouvement de l'élément d'actionnement de la partie d'entrée d'opération de torsion (20, 20B), la partie de saillie (43Bf, 43b, 43Ab, 43Bb) est insérée dans la partie de rainure (42Bf) ou détachée de celle-ci ;
dans lequel la partie de saillie (43Bf, 43b, 43Ab, 43Bb) est formée au niveau d'une partie correspondant à la partie de rainure (42Bf) de l'élément en forme de plaque (42, 42B) lorsque l'élément en forme de plaque (42, 42B) est reçu à l'intérieur de la partie de logement (43, 43A, 43B, 45, 45A, 45B) dans un état prédéterminé.

2. Dispositif endoscopique tordu électriquement selon la revendication 1, dans lequel l'élément en forme de plaque (42, 42B) est de forme sensiblement circulaire.

3. Dispositif endoscopique tordu électriquement selon la revendication 2,
dans lequel le corps de logement (43, 43A, 43B, 45, 45A, 45B) présente une forme creuse et sensiblement cylindrique comportant un espace interne (44) supérieur à l'élément en forme de plaque (42, 42B) et une plage de déplacement de l'élément en forme de plaque (42, 42B), et
dans lequel, dans l'espace interne (44), l'élément en forme de plaque (42, 42B) est reçu et le fluide visqueux (44a) est contenu de manière étanche.

4. Dispositif endoscopique tordu électriquement selon la revendication 2,
dans lequel le corps de logement (43, 43A, 43B, 45, 45A, 45B) présente une forme creuse et sensiblement quadrangulaire comportant un espace interne (44A) supérieur à l'élément en forme de plaque (42, 42B) et une plage de déplacement de l'élément en forme de plaque (42, 42B), et
dans lequel, dans l'espace interne (44A), l'élément en forme de plaque (40, 42B) est reçu et le fluide visqueux (44a) est contenu de manière étanche.

5. Dispositif endoscopique tordu électriquement selon l'une quelconque des revendications 1 à 4, dans lequel la partie d'entrée d'opération de torsion (20, 20B) est un type de manche à balai.

6. Dispositif endoscopique tordu électriquement selon la revendication 5, comprenant en outre :
un élément de régulation (47, 47D) destiné à réguler le mouvement de l'élément d'actionnement (41) de la partie d'entrée d'opération de torsion (20, 20B).

7. Endoscope tordu électriquement selon la revendication 1,
dans lequel l'élément d'actionnement (41) de la partie d'entrée d'opération de torsion (20, 20B) présente une forme de levier inclinable dans une pluralité de directions avec pour référence une position neutre prédéterminée, et
l'élément en forme de plaque (42, 42B) est prévu dans une direction sensiblement orthogonale à une direction axiale (41a) de l'élément d'actionnement (41) dans un état, dans lequel il est disposé au niveau de la position neutre d'une manière telle que l'élément en forme de plaque (42, 42B) peut se déplacer dans la pluralité de directions en liaison avec le mouvement de l'élément d'actionnement (41).
